# EUROPÄISCHE PATENTANMELDUNG

(11) **EP 1 149 630 A2**
(43) Veröffentlichungstag der Anmeldung: **31.10.2001**
(21) Anmeldenummer: 01109538.7
(22) Anmeldetag: 17.04.2001
(51) Int. Cl.: B01J 39/20, C02F 1/42, C07C 37/20, B01J 31/10

(54) **Verfahren zur Herstellung von monodispersen gelförmigen Kationenaustauschern**

(30) Priorität: 27.04.2000 DE 10020534
(71) Anmelder: Bayer Aktiengesellschaft, 51368 Leverkusen (DE)
(72) Erfinder: Schmid, Claudia, Dr., 42799 Leichlingen (DE); Podszun, Wolfgang, Dr., 51061 Köln (DE); Seidel, Rüdiger, Dr., 51375 Leverkusen (DE); Halle, Olaf, Dr., 51061 Köln (DE); Born, Ralf-Jürgen, Dr., 40764 Langenfeld (DE); Klipper, Reinhold, Dr., 50933 Köln (DE)

(57) **Zusammenfassung**

Die vorliegende Erfindung betrifft ein Verfahren zur Herstellung monodisperser gelförmiger Kationenaustauscher mit erhöhter Oxidationsstabilität und hoher osmotischer Stabilität und Reinheit, welche erhalten werden, indem man
a) monodisperse microverkapselte Monomertröpfchen aus einem Monomergemisch 1 enthaltend 90,5 bis 97,99 Gew.-% Styrol, 2 bis 7 Gew.-% Divinylbenzol und 0,01 bis 2,5 Gew.-% Radikalbildner in wässriger Suspension bis zu Umsätzen von 76 bis 100 % polymerisiert,
b) ein Monomergemisch 2 aus 70,5 bis 95,99 Gew.-% Styrol, 4 bis 15 Gew.-% Divinylbenzol, 0 bis 12 Gew.-% eines dritten Comonomers und 0,01 bis 2,5 Gew.-% eines oder mehrerer Radikalbildner unter Bildung eines Copolymerisates zusetzt, wobei man eine Teilmenge von 50 bis 100 Gew.-% des Monomergemisches 2 unter polymerisierenden Bedingungen zusetzt, unter denen mindestens ein Radikalbildner aus Monomergemisch 2 aktiv ist, und schließlich
c) das Reaktionsprodukt aus Verfahrensschritt b) durch Sulfonierung funktionalisiert,
die Kationenaustauscher selber sowie deren Verwendungen.

## Beschreibung

Die Erfindung betrifft ein Verfahren zur Herstellung von monodispersen gelförmigen Kationenaustauschern mit hoher Oxidationsstabilität sowie hoher osmotischer Stabilität und Reinheit.

In jüngster Zeit haben Ionenaustauscher mit möglichst einheitlicher Teilchengröße (im folgenden "monodispers" genannt) zunehmend an Bedeutung gewonnen, weil bei vielen Anwendungen aufgrund der günstigeren hydrodynamischen Eigenschaften eines Austauscherbettes aus monodispersen Ionenaustauschern wirtschaftliche Vorteile erzielt werden können. Monodisperse Ionenaustauscher können durch Funktionalisieren von monodispersen Perlpolymerisaten erhalten werden.

Eine der Möglichkeiten, monodisperse Perlpolymerisate herzustellen, besteht im sog. seed/feed-Verfahren, wonach ein monodisperses Polymerisat ("Saat") im Monomer gequollen und dieses dann polymerisiert wird. Seed/feed-Verfahren werden beispielsweise in den EP-A 0 098 130 und EP-A 0 101 943 beschrieben.

Die EP-A 0 826 704 offenbart ein Seed/feed-Verfahren, bei welchem mikroverkapseltes vernetztes Perlpolymerisat als Saat eingesetzt wird.

Ein Problem der bekannten Kationenaustauscher besteht darin, dass diese aufgrund von ursprünglich vorhandenen oder während des Gebrauchs gebildeten löslichen Polymeren zu unerwünschtem Ausbluten (leaching) neigen können.

In der DE-A 19 852 667 wird deshalb ein Verfahren zur Herstellung von monodispersen gelförmigen Kationenaustauschern beschrieben, mit dem diese eine höhere Stabilität und Reinheit erhalten werden. Nachteilig an dem Verfahren gemäß DE-A 19 852 667 ist die Tatsache, dass es sich dabei um cin sogenanntes statisches seed/feed Verfahren handelt, bei dem eine niedrig vernetzte Saat eingesetzt wird und man durch Zugabe des Feeds unter nichtpolymerisierenden Bedingungen eine uneinheitliche Netzbogenlängenverteilung im Perlpolymerisat und somit auch im Kationenaustauscher erhält. Dies mindert beispielsweise die Oxidationsstabilität der Harze, sobald sie beispielsweise in Vollentsalzungsanlagen eingesetzt werden.

Ein weiteres Problem der bekannten Kationenaustauscher besteht in ihrer nicht immer ausreichenden mechanischen und osmotischen Stabilität. So können Kationenaustauscherperlen bei der Verdünnung nach der Sulfonierung durch die auftretenden osmotischen Kräfte zerbrechen. Für alle Anwendungen von Kationenaustauschern gilt, dass die in Perlform vorliegenden Austauscher ihren Habitus behalten müssen und nicht während der Anwendung teilweise oder auch gänzlich abgebaut werden oder in Bruchstücke zerfallen dürfen. Bruchstücke und Perlpolymerisatsplitter können während der Reinigung in die zu reinigenden Lösungen gelangen und diese selbst verunreinigen. Ferner ist das Vorhandensein von geschädigten Perlpolymerisaten für die Funktionsweise der in Säulenverfahren eingesetzten Kationenaustauscher selbst ungünstig. Splitter führen zu einem erhöhten Druckverlust des Säulensystems und vermindern damit den Durchsatz an zu reinigender Flüssigkeit durch die Säule.

Für Kationenaustauscher gibt es eine Vielzahl unterschiedlicher Anwendungen. So werden sie beispielsweise bei der Trinkwasseraufbereitung, bei der Herstellung von Reinstwasser (notwendig bei der Mikrochip-Herstellung für die Computerindustrie), zur chromatographischen Trennung von Glucose und Fructose oder als Katalysatoren für verschiedene chemische Reaktionen (wie z.B. bei der Bisphenol-A-Herstellung aus Phenol und Aceton) eingesetzt. Für die meisten dieser Anwendungen ist erwünscht, dass die Kationenaustauscher die ihnen zugedachten Aufgaben erfüllen, ohne Verunreinigungen, die von ihrer Herstellung herrühren können oder während des Gebrauches durch Polymerabbau entstehen, an ihre Umgebung abzugeben. Das Vorhandensein von Verunreinigungen im vom Kationenaustauscher abfließenden Wasser macht sich dadurch bemerkbar, dass der pH-Wert abfällt und die Leitfähigkeit und/oder der Gehalt an organischem Kohlenstoff (TOC Gehalt) des Wassers erhöht sind.

Die Aufgabe der vorliegenden Erfindung ist deshalb die Bereitstellung von monodispersen gelförmigen Kationenaustauschern mit einerseits hoher Stabilität und Reinheit, aber andererseits auch mit einheitlicheren Netzbogenlängenverteilungen und somit einer verbesserten Oxidationsstabilität gegenüber den Kationenaustauschern, wie sie aus dem Stand der Technik her bekannt sind.

Unter Reinheit im Sinne der vorliegenden Erfindung ist in erster Linie gemeint, dass die Kationenaustauscher nicht ausbluten. Das Ausbluten äußert sich in einem Anstieg der Leitfähigkeit des Wassers, das mit dem Ionenaustauscher behandelt wird.

Die Lösung der Aufgabe und damit der Gegenstand der vorliegenden Erfindung ist ein Verfahren zur Herstellung von monodispersen gelförmigen Kationenaustauschern mit erhöhter Oxidationsstabilität und hoher osmotischer Stabilität und Reinheit, dadurch gekennzeichnet, dass man
a) monodisperse microverkapselte Monomertröpfchen aus einem Monomergemisch 1 enthaltend 90,5 bis 97,99 Gew.-% Styrol, 2 bis 7 Gew.-% Divinylbenzol und 0,01 bis 2,5 Gew.-% Radikalbildner in wässriger Suspension bis zu Umsätzen von 76 bis 100 % polymerisiert,
b) ein Monomergemisch 2 aus 70,5 bis 95,99 Gew.-% Styrol, 4 bis 15 Gew.-% Divinylbenzol, 0 bis 12 Gew.-% eines dritten Comonomers und 0,01 bis 2,5 Gew.-% eines oder meherer Radikalbildner unter Bildung eines Copolymerisates zusetzt, wobei man eine Teilmenge von 50 bis 100 Gew.-% des Monomergemisches 2 unter polymerisierenden Bedingungen zusetzt, unter denen mindestens ein Radikalbildner aus Monomergemisch 2 aktiv ist, und schließlich
c) das Reaktionsprodukt aus Verfahrensschritt b) durch Sulfonierung funktionalisiert.

In einer bevorzugten Ausführung des erfindungsgemäßen Verfahrens wird nach dem Verfahrensschritt b) in einem Zwischenschritt b') der Polymerisationsumsatz der Monomerengemische 1 und 2 erhöht bevor das Copolymerisat schließlich durch Sulfonierung funktionalisiert wird.

Um die monodisperse Eigenschaft der Produkte zu gewährleisten, erfolgt die Zugabe des Monomergemisches 2 durch Jetten, seed/feed oder Direktverdüsung. Diese Verfahren sind bekannt aus US-A 3 922 255, US-A 4 444 961 und US-A 4 427 794.

Im Rahmen der vorliegenden Erfindung kann im Verfahrensschritt a) das Divinylbenzol in kommerziell verfügbarer Qualität, welches neben den Isomeren des Divinylbenzols auch Ethylvinylbenzol enthält, eingesetzt werden. Die Menge an reinem Divinylbenzol beträgt im Rahmen der vorliegenden Erfindung 2 bis 7 Gew.-%, vorzugsweise 3 bis 6 Gew.-% bezogen auf das Monomergemisch 1.

Als Radikalbildner kommen im Verfahrensschritt a) im Rahmen der vorliegenden Erfindung beispielsweise Peroxyverbindungen wie Dibenzoylperoxid, Dilauroylperoxid, Bis(p-chlorbenzoyl)peroxid, Dicyclohexylperoxydicarbonat, tert.-Butylperoctoat, 2,5-Bis(2-ethylhexanoylperoxy)-2,5-dimethylhexan oder tert.-Amylperoxy-2-ethylhexan, desweiteren Azoverbindungen wie 2,2'-Azobis(isobutyronitril) oder 2,2'-Azobis(2-methylisobutyronitril) in Frage. Gut geeignet sind auch aliphatische Peroxyester.

Beispiele für aliphatische Peroxyester entsprechen den Formeln (I), (II) oder (III): 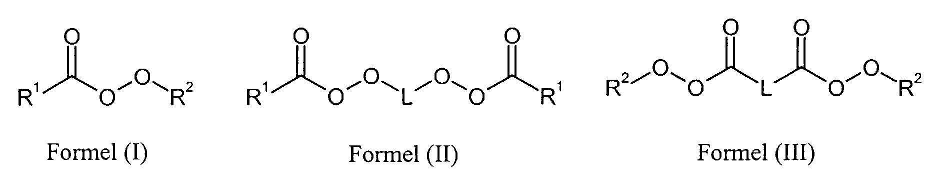 worin
- R¹: für einen Alkylrest mit 2 bis 20 C-Atomen oder einen Cycloalkylrest mit bis zu 20 C-Atomen steht,
- R²: für einen verzweigten Alkylrest mit 4 bis 12 C-Atomen steht und
- L: für einen Alkylrest mit 2 bis 20 C-Atomen oder einen Cycloalkylenrest mit bis zu 20 C-Atomen steht.

Erfindungsgemäß bevorzugte aliphatische Peroxyester gemäß Formel (I) sind beispielsweise
tert.-Butylperoxyacetat,
tert.-Butylperoxyisobutyrat,
tert.-Butylperoxypivalat,
tert.-Butylperoxyoctoat,
tert.-Butylperoxy-2-ethylhexanoat,
tert.-Butylperoxyneodecanoat,
tert.-Amylperoxyneodecanoat,
tert.-Amylperoxypivalat,
tert.-Amylperoxyoctoat oder
tert.-Amylperoxy-2-ethylhexanoat.

Bevorzugte aliphalische Peroxyester gemäß Formel (II) sind beispielsweise
2,5-Bis(2-ethylhexanoylperoxy)-2,5-dimethylhexan,
2,5-Dipivaloyl-2,5-dimethylhexan oder
2,5-Bis(2-neodecanoylperoxy)-2,5-dimethylhexan.

Bevorzugte aliphatische Peroxyester gemäß Formel (III) sind beispielsweise
Di-tert.-butylperoxyazelat oder
Di-tert.-amylperoxyazelat.

Erfindungsgemäß werden die Radikalbildner im Verfahrensschritt a) im allgemeinen in Mengen von 0,01 bis 2,5, vorzugsweise 0,1 bis 1,5 Gew.-% bezogen auf Monomerengemisch 1 im Verfahrensschritt a) angewendet. Selbstverständlich können auch Mischungen der vorgenannten Radikalbildner eingesetzt werden, beispielsweise Mischungen von Radikalbildnern mit unterschiedlichen Zerfallstemperaturen.

Für die Mikroverkapselung der Monomertröpfchen im Verfahrensschritt a) kommen die für diesen Einsatzzweck bekannten Materialien in Frage, insbesondere Polyester, natürliche und synthetische Polyamide, Polyurethane oder Polyhamstoffe. Als natürliches Polyamid ist Gelatine besonders gut geeignet. Diese kommt insbesondere als Koazervat oder Komplexkoazervat zur Anwendung. Unter gelatinehaltigen Komplexkoazervaten im Sinne der Erfindung werden vor allem Kombinationen von Gelatine und synthetischen Polyelektrolyten verstanden. Geeignete synthetische Polyelektrolyte sind Copolymerisate mit eingebauten Einheiten von beispielsweise Maleinsäure, Acrylsäure, Methacrylsäure, Acrylamid oder Methacrylamid. Gelatinehaltige Kapseln können mit üblichen Härtungsmitteln wie beispielsweise Formaldehyd oder Glutardialdehyd gehärtet werden. Die Verkapselung von Monomertröpfchen beispielsweise mit Gelatine, gelatinehaltigen Koazervaten oder gelatinehaltigen Komplexkoazervaten, wird in EP-A 0 046 535 eingehend beschrieben. Die Methoden der Verkapselung mit synthetischen Polymeren sind bekannt. Gut geeignet ist beispielsweise die Phasengrenzflächenkondensation, bei der eine im Monomertröpfchen gelöste Reaktivkomponente (z.B. ein Isocyanat oder ein Säurechlorid) mit einer zweiten in der wässrigen Phase gelösten Reaktivkomponente (z.B. einem Amin) zur Reaktion gebracht wird. Die Mikroverkapselung mit gelatinehaltigem Komplexkoazervat ist bevorzugt.

Die Polymerisation der monodispersen microverkapselten Tröpfchen aus Monomergemisch 1 im Verfahrensschritt a) erfolgt in wässriger Suspension bei erhöhter Temperatur von z.B. 55 bis 95°C, vorzugsweise 60 bis 80°C und bis zu einem Umsatz von 76 bis 100 Gew.-%, vorzugsweise 85 bis 100 Gew.-%. Die jeweils günstigste Polymerisationstemperatur ergibt sich für den Fachmann aus den Halbwertszeiten der Radikalbildner. Bei der Polymerisation wird die Suspension gerührt. Die Rührgeschwindigkeit ist dabei unkritisch. Es können niedrige Rührgeschwindigekiten angewendet werden, die gerade ausreichen, um die suspendierten Teilchen in Schwebe zu halten.

Das Massenverhältnis von Monomergemisch 1 zu Wasser beträgt 1 : 1 bis 1 : 20, vorzugsweise 1 : 2 bis 1 : 10.

Zur Stabilisierung der mikroverkapselten Monomertröpfchen in der Wasserphase können Dispergierhilfsmittel eingesetzt werden. Erfindungsgemäß geeignete Dispergierhilfsmittel sind natürliche und synthetische wasserlösliche Polymere, wie z.B. Gelatine, Stärke, Polyvinylalkohol, Polyvinylpyrrolidon, Polyacrylsäure, Polymethacrylsäure oder Copolymerisate aus (Meth)acrylsäure oder (Meth)acrylsäureestern. Sehr gut geeignet sind auch Cellulosederivate, insbesondere Celluloseester oder Celluloseether, wie Carboxymethylcellulose oder Hydroxyethylcellulose. Die Einsatzmenge der Dispergierhilfsmittel beträgt im allgemeinen 0,05 bis 1 Gew.-% bezogen auf die Wasserphase, vorzugsweise 0,1 bis 0,5 Gew.-%.

Die Polymerisation in Verfahrensschritt a) kann gegebenenfalls in Anwesenheit eines Puffersystems durchgeführt werden. Erfindungsgemäß bevorzugte Puffersysteme stellen den pH-Wert der Wasserphase bei Beginn der Polymerisation auf einen Wert zwischen 12 und 3, vorzugsweise zwischen 10 und 4 ein. Besonders gut geeignete Puffersysteme enthalten Phosphat-, Acetat-, Citrat- oder Boratsalze.

Es kann vorteilhaft sein, einen in der wässrigen Phase gelösten Inhibitor einzusetzen. Als Inhibitoren kommen sowohl anorganische als auch organische Stoffe in Frage. Beispiele für anorganische Inhibitoren sind Stickstoffverbindungen wie Hydroxylamin, Hydrazin, Natriumnitrit oder Kaliumnitrit. Beispiele für organische Inhibitoren sind phenolische Verbindungen wie Hydrochinon, Hydrochinonmonomethylether, Resorcin, Brenzkatechin, tert.-Butylbrenzkatechin oder Kondensationsprodukte aus Phenolen mit Aldehyden. Weitere organische Inhibitoren sind stickstoffhaltige Verbindungen wie z.B. Diethylhydroxylamin oder Isopropylhydroxylamin. Erfindungsgemäß wird Resorzin als Inhibitor bevorzugt. Die Konzentration des Inhibitors beträgt 5 bis 1000 ppm, vorzugsweise 10 bis 500 ppm, besonders bevorzugt 20 bis 250 ppm, bezogen auf die wässrige Phase.

In einer besonderen Ausführungsform des Verfahrensschrittes a) der vorliegenden Erfindung enthält die wässrige Suspension in der wässrigen Phase gelöstes Acrylnitril. Die Menge an Acrylnitril beträgt 1 bis 10 Gew.-%, bevorzugt 2 bis 8 Gew.-% bezogen auf Monomergemisch 1.

Es wurde gefunden, dass das der wässrigen Phase zugesetzte Acrylnitril in hohen Anteilen in das Polymerisat aus dem Monomergemisch 1 einpolymerisiert. Die Einbaurate des Acrylnitrils beträgt beim erfindungsgemäßen Verfahren mehr als 90 Gew.-%, vorzugsweise mehr als 95 Gew.-%.

Die Teilchengröße der monodispersen mikroverkapselten Monomertröpfchen in Verfahrensschritt a) beträgt 10 bis 600 µm, vorzugsweise 20 bis 450 µm, besonders bevorzugt 100 bis 400 µm. Zur Bestimmung der mittleren Teilchengröße und der Teilchengrößenverteilung sind übliche Methoden, wie Siebanalyse oder Bildanalyse geeignet. Als Maß für die Breite der Teilchengrößenverteilung der Monomertröpfchen wird das Verhältnis aus dem 90 %-Wert (Ø (90)) und dem 10 %-Wert (Ø (10)) der Volumenverteilung gebildet. Der 90 %-Wert (Ø (90)) gibt den Durchmesser an, der von 90 % der Teilchen unterschritten wird. In entsprechender Weise unterschreiten 10 % der Teilchen den Durchmesser des 10 %-Wertes (Ø (10)). Monodisperse Teilchengrößenverteilungen im Sinne der Erfindung bedeuten Ø (90)/Ø (10) ≤ 1,5, vorzugsweise Ø (90)/Ø (10) ≤ 1,25.

Die aus Verfahrensschritt a) resultierende Polymersuspension kann direkt in Verfahrensschritt b) weiterverarbeitet werden. Es ist auch möglich, das Polymerisat aus Verfahrensschritt a) zu isolieren, falls gewünscht, zu waschen, zu trocknen und zwischenzulagern.

Sofern das unter Verfahrensschritt a) erhaltene Polymerisat isoliert wurde, wird es in Verfahrensschritt b) in wässriger Phase suspendiert, wobei das Massenverhältnis von Polymerisat und Wasser zwischen 1 : 1 und 1 : 20 liegen kann. Erfindungsgemäß bevorzugt wird ein Massenverhältnis von 1:1 bis 1: 10. Die Wasserphase enthält ein Dispergierhilfsmittel, wobei die Art und Menge des Dispergierhilfsmittels den oben unter Verfahrensschritt a) genannten entsprechen kann.

Das Monomergemisch 2 in Verfahrensschritt b) enthält 4 bis 15 Gew.-%, vorzugsweise 5 bis 12 Gew.-% reines Divinylbenzol. Wie bereits oben unter Verfahrensschritt a) beschrieben, können technische Divinylbenzolqualitäten verwendet werden.

Beispiele für ein drittes Comonomer in Monomergemisch 2 sind Ester der Acrylsäure oder Methacrylsäure wie Methylmethacrylat, Methylacrylat oder Ethylacrylat sowie Acrylnitril oder Methacrylnitril. Acrylnitril ist bevorzugt. Die Menge an drittem Comonomer beträgt 0 bis 12 Gew.-%, vorzugsweise 2 bis 10 Gew.-%, besonders bevorzugt 4 bis 8 Gew.-% bezogen auf das Monomergemisch 2.

Als Radikalbildner kommen im Verfahrensschritt b) die unter Verfahrensschritt a) beschriebenen Radikalbildner in Frage. Auch im Verfahrensschritt b) sind aliphatische Peroxyester bevorzugt.

Das Massenverhältnis von Polymerisat aus Verfahrenschritt a) zu Monomergemisch 2 beträgt 1:0,5-1 : 10, vorzugsweise 1:0,75 - 1:6. Die Zugabe des Monomergemisches 2 zu dem im Verfahrensschritt a) erhaltenen Polymerisat erfolgt in der Weise, dass eine erste Teilmenge von 0 - 50 Gew.-%, vorzugsweise 10-50 Gew.-%, besonders bevorzugt 10-25 Gew.-% unter Bedingungen zugesetzt wird, unter denen keines der Radikalbildner aus Monomergemisch 2 aktiv ist, im Allgemeinen bei einer Temperatur von 0-50°C, vorzugsweise von 10-40°C.

Die Zugabe der zweiten Teilmenge des Monomergemisches 2, die sich mit der ersten Teilmenge zu 100% ergänzt, erfolgt über einen längeren Zeitraum z.B. über 10-1000 min, vorzugsweise über 30-600 min unter Bedingungen, unter denen mindestens ein Radikalbildner aus Monomergemisch 2 aktiv ist.

Die Zugabe kann dabei mit gleichbleibender Geschwindigkeit oder mit einer über die Zeit veränderten Geschwindigkeit erfolgen. Die Zusammensetzung des Monomergemisches 2 kann innerhalb der vorgesehenen Grenzen während der Zugabe verändert werden. Weiterhin ist es möglich, dass die erste Teilmenge und die zweite Teilmenge sich in der Zusammensetzung in Bezug auf Gehalt an Divinylbenzol, Comonomer oder Radikalbildner unterscheiden.

Die Temperatur während der Zugabe wird so gewählt, dass mindestens einer der im System vorhandenen Radikalbildner aktiv ist. Im Allgemeinen werden Temperaturen von 60 bis 130°C, vorzugsweise 60 bis 95°C angewendet. Nach Abschluss von Verfahrensschritt b) beträgt der Polymerisationsumsatz der Monomergemische 1 und 2 im Allgemeinen 60 - 95 Gew.-%.

Das Monomergemisch 2 gemäß der vorliegenden Erfindung kann in reiner Form zugesetzt werden. In einer besonderen Ausführungsform wird das Monomergemisch 2 oder ein Teil dieses Gemisches in Form einer Emulsion in Wasser zugesetzt. Diese Emulsion in Wasser kann in einfacher Weise durch Vermischen des Monomergemisches mit Wasser unter Anwendung eines Emulgiermittels beispielsweise mit Hilfe eines Schnellrührers, eines Rotor-Stator-Mischers oder einer Nassstrahldüse erzeugt werden. Das Massenverhältnis von Monomergemisch zu Wasser beträgt dabei vorzugsweise 1 : 0,75 bis 1 : 3. Die Emulgiermittel können ionischer oder nichtionischer Natur sein. Gut geeignet sind beispielsweise ethoxylierte Nonylphenole mit 2 bis 30 Ethylenoxideinheiten, aber auch das Natriumsalz des Sulfobernsteinsäureisooctylesters.

Im gegebenenfalls nach Verfahrensschritt b) einzufügenden Zwischenschritt b') gemäß der bevorzugten Ausführungsform der vorliegenden Erfindung wird zur gegebenenfalls vorteilhaften Vervollständigung des Polymerisationsumsatzes der Monomergemische 1 und 2 das Polymerisationsgemisch nach beendeter Zugabe des Monomergemisches 2 über einen Zeitraum von 1 bis 8 h bei einer Temperatur von 60 bis 140°C, vorzugsweise 90 bis 130°C gehalten. Zur Erzielung hoher Polymerisationsumsätze ist es günstig, die Temperatur während der Auspolymerisation zu steigern. Durch den Verfahrensschritt b') wird der Polymerisationsumsatz der Monomergemische 1 und 2 auf 90 - 100 Gew.-%, vorzugsweise auf 95 - 100 Gew.-% gesteigert.

Nach der Polymerisation kann das daraus resultierende Copolymerisat mit den üblichen Methoden z.B. durch Filtrieren oder Dekantieren isoliert und gegebenenfalls nach einer oder mehreren Wäschen mit entionisiertem Wasser getrocknet und falls gewünscht gesiebt werden.

Die Umsetzung des Reaktionsproduktes aus Verfahrensschritt b) bzw. des Reaktionsproduktes aus dem nach Verfahrensschritt b) eingefügten Zwischenschritt b') zum Kationenaustauscher gemäß Verfahrensschritt c) erfolgt durch Sulfonierung. Geeignete Sulfonierungsmittel im Rahmen der vorliegenden Erfindung sind Schwefelsäure, Schwefeltrioxid und Chlorsulfonsäure. Bevorzugt wird Schwefelsäure mit einer Konzentration von 90 bis Gew.-100 %, besonders bevorzugt von 96 bis 99 Gew.-%. Die Temperatur bei der Sulfonierung liegt im allgemeinen bei 50 bis 200°C, bevorzugt bei 90 bis 150°C, besonders bevorzugt bei 95 bis 130°C. Es wurde gefunden, dass die erfindungsgemäßen Copolymerisate ohne Zusatz von Quellungsmitteln (wie z.B. Chlorbenzol oder Dichlorethan) sulfoniert werden können und dabei homogene Sulfonierungsprodukte liefern.

Bei der Sulfonierung wird das Reaktionsgemisch gerührt. Dabei können verschiedene Rührertypen, wie Blatt-, Anker-, Gitter- oder Turbinenrührer eingesetzt werden. Es hat sich gezeigt, dass ein radialfördernder Doppelturbinenrührer besonders gut geeignet ist.

In einer besonderen Ausführungsform der vorliegenden Erfindung erfolgt die Sulfonierung nach dem sogenannten "Semibatchverfahren". Bei dieser Methode wird das Copolymerisat in die temperierte Schwefelsäure eindosiert. Dabei ist es besonders vorteilhaft, die Dosierung portionsweise durchzuführen.

Nach der Sulfonierung wird das Reaktionsgemisch aus Sulfonierungsprodukt und Restsäure auf Raumtemperatur abgekühlt und zunächst mit Schwefelsäuren abnehmender Konzentrationen und dann mit Wasser verdünnt.

Falls gewünscht kann der erfindungsgemäß erhaltene Kationenaustauscher in der H-Form zur Reinigung mit entionisiertem Wasser bei Temperaturen von 70 bis 145°C, vorzugsweise von 105 bis 130°C behandelt werden.

Für viele Anwendungen ist es günstig, den Kationenaustauscher von der sauren Form in die Natrium-Form zu überführen. Diese Umladung erfolgt mit Natronlauge einer Konzentration von 10 bis 60 Gew.-%, vorzugsweise 40 bis 50 Gew.-%. Die Umladung kann bei einer Temperatur von 0 bis 120°C, beispielsweise bei Raumtemperatur, durchgeführt werden. Bei diesem Verfahrensschritt kann die auftretende Reaktionswärme zur Einstellung der Temperatur genutzt werden.

Nach der Umladung können die Kationenaustauscher zur weiteren Reinigung mit entionisiertem Wasser oder wässrigen Salzlösungen, beispielsweise mit Natriumchlorid- oder Natriumsulfatlösungen, behandelt werden. Dabei wurde gefunden, dass die Behandlung bei 70 bis 150°C, vorzugsweise 120 bis 135°C besonders effektiv ist und keine Verringerung der Kapazität des Kationenaustauschers bewirkt.

Die nach dem erfindungsgemäßen Verfahren erhaltenen Kationenaustauscher weisen eine hohe Monodispersität auf. Die Teilchenverteilung der Kationenaustauscher ist ein vergrößertes Abbild der Teilchenverteilung der microverkapselten Monomertröpfchen. Es ist überraschend, dass trotz der Microverkapselung der Monomertröpfchen das im Verfahrensschritt b) zugesetzte Monomergemisch vollständig und gleichmäßig in die im Verfahrensschritt a) gebildeten Polymerisatpartikel eindringt.

Die erhaltenen Kationenaustauscher zeichnen sich durch eine besonders hohe Stabilität und Reinheit aus. Sie zeigen auch nach längerem Gebrauch und vielfacher Regeneration äußerst wenig Defekte an den Ionenaustauscherkugeln und ein im Vergleich mit den Produkten des Standes der Technik verringertes Ausbluten (Leaching) des Austauschers.

Aufgrund ihrer gegenüber dem Stand der Technik deutlich erhöhten Stabilität und Reinheit, insbesondere der erhöhten Oxidationsstabilität eignen sich die Kationenaustauscher gemäß der vorliegenden Erfindung insbesondere zur Trinkwasseraufbereitung, zur Herstellung von Reinstwasser, zur chromatografischen Trennung von Zuckern, beispielsweise von Glucose und Fructose, sowie als Katalysatoren für chemische Reaktionen, bei Kondensationsreaktionen, insbesondere bei der Bisphenol-A-Synthese aus Phenol und Aceton.

Weiterhin eignen sich die erfindungsgemäßen Kationenaustauscher
- zur Entfernung von Kationen, Farbpartikeln oder organischen Komponenten aus wässrigen oder organischen Lösungen und Kondensaten, wie z.B. Prozess- oder Turbinenkondensaten.
- zur Enthärtung im Neutralaustausch von wässrigen oder organischen Lösungen und Kondensaten, wie z.B. Prozess- oder Turbinenkondensaten.
- zur Reinigung und Aufarbeitung von Wässern der chemischen Industrie, der Elektronik-Industrie und aus Kraftwerken,
- zur Vollentsalzung wässriger Lösungen und/oder Kondensate, wie z.B. Prozess- oder Turbinenkondensaten,
- in Kombination mit heterodispersen oder monodispersen, gelförmigen und/oder makroporösen Anionenaustauschern zur Vollentsalzung wässriger Lösungen und/oder Kondensate, wie z.B. Prozess- oder Turbinenkondensate,
- zur Entfärbung und Entsalzung von Molken, Gelatinedünnbrühen, Fruchtsäften, Fruchtmosten und wässrigen Lösungen von Zuckern.

### Infolgedessen sind ebenfalls Gegenstand der Erfindung

Verfahren zur Entfernung von Kationen, Farbpartikeln oder organischen Komponenten aus wässrigen oder organischen Lösungen und Kondensaten, wie z.B. Prozess- oder Turbinenkondensaten unter Einsatz der erfindungsgemäßen Kationenaustauscher.

Verfahren zur Erhärtung im Neutralaustausch von wässrigen oder organischen Lösungen und Kondensaten, wie z.B. Prozess- oder Turbinenkondensaten unter Einsatz erfindungsgemäßer Kationenaustauscher.

Verfahren zur Reinigung und Aufarbeitung von Wässern der chemischen, der Elektronik-Industrie und aus Kraftwerken unter Einsatz der erfindungsgemäßen Kationenaustauscher.

Verfahren zur Vollentsalzung wässriger Lösungen und/oder Kondensate, wie z.B. Prozess- oder Turbinenkondensate unter Einsatz der erfindungsgemäßen Kationenaustauscher in Kombination mit heterodispersen oder monodispersen, gelförmigen und/oder makroporösen Anionenaustauschern.

Verfahren zur Entfärbung und Entsalzung von Molken, Gelatinedünnbrühen, Fruchtsäften, Fruchtmosten und wässrigen Lösungen von Zuckern in der Zucker-, Stärke- oder Pharmaindustrie oder Molkereien unter Einsatz der erfindungsgemäßen Kationenaustauscher.

### Beispiele

### Beispiel 1 (erfindungsgemäß)

### a) Herstellung eines Copolymerisates

1260 g eines wässrigen Gemisches enthaltend 630 g monodisperse microverkapselte Monomertröpfchen mit einer mittleren Teilchengröße von 330 µm und einem ⌀ (90)/⌀ (10)-Wert von 1,03, bestehend aus 94,53 Gew.-% Styrol, 4,98 Gew.-% Divinylbenzol und 0,50 Gew.-% tert-Butyl-peroxy-2-ethylhexanoat werden mit einer wässrigen Lösung aus 2,13 g Gelatine, 3,52 g Natriumhydrogenphosphatdodekahydrat und 175 mg Resorcin in 1400 ml entionisiertem Wasser in einem 4 1 Glasreaktor versetzt. Die Mischung wird unter Rühren (Rührgeschwindigkeit 200 Upm) 8 h bei 75°C und anschließend 2 h bei 95°C polymerisiert. Der Ansatz wird über ein 32 µm-Sieb gewaschen und getrocknet. Man erhält 622 g eines Perlpolymerisats mit glatter Oberfläche. Die Polymerisate erscheinen optisch transparent.

600,0 g eines monodispersen mikroverkapselten Polymerisats, hergestellt nach obigem Verfahren, werden mit einer wässrigen Lösung von 3,58 g Borsäure und 0,98 g Natriumhydroxid in 1100,0 g entionisiertem Wasser versetzt. Zu dieser Mischung wird unter Rühren (Rührgeschwindigkeit 220 Upm) ein Gemisch aus 93,4 g Styrol, 17,0 g 80,6 %-igem Divinylbenzol, 9,6 g Acrylnitril, 0,43 g tert-Butyl-peroxy-2-ethylhexanoat und 0,30 g tert-Butyl-peroxybenzoat (Zugabegeschwindigkeit: 4,0 g/min) gegeben. Nach einer Einquellzeit von 1 h wird das Gemisch mit einer wässrigen Lösung von 2,44 g Methylhydroxyethylcellulose (Walocel MT 400® ) in 122 g entionisiertem Wasser versetzt. Die Mischung wird 10 h bei 63°C polymerisiert. Unmittelbar nach Erreichen der Polymerisationstemperatur von 63°C wird ein Monomergemisch aus 373,7 g Styrol, 67,9 g 80,6 %-igem Divinylbenzol, 38,4 g Acrylnitril, 1,73 g tert-Butyl-peroxy-2-ethylhexanoat und 1,20 g tert-Butylperoxybenzoat über einen Zeitraum von 5 h mit einer gleichbleibenden Geschwindigkeit zugetropft. Anschließend wird der Ansatz 3 h bei 130°C gehalten. Der Ansatz wird über ein 32 µm-Sieb gewaschen und getrocknet. Man erhält 1186 g eines Perlpolymerisats mit glatter Oberfläche. Die Polymerisate erscheinen optisch transparent; die mittlere Teilchengröße beträgt 410 µm und der ⌀ (90)/⌀ (10)-Wert 1,06.

### b) Herstellung eines Kationenaustauschers

In einem 2 1 Vierhalskolben werden 1800 ml 97,32 Gew.-%ige Schwefelsäure vorgelegt und auf 100°C erhitzt. In 4 h werden in 10 Portionen - insgesamt 400 g trockenes Polymerisat aus Beispiel 1a, unter Rühren eingetragen. Anschließend wird weitere 6 h bei 120°C gerührt. Nach dem Abkühlen wird die Suspension in eine Glassäule überführt. Schwefelsäuren abnehmender Konzentrationen, beginnend mit 90 Gew.-%, zuletzt reines Wasser werden von oben über die Säule filtriert. Man erhält 1792 ml Kationenaustauscher in der H-Form.

### Beispiel 2 (erfindungsgemäß)

### a) Herstellung eines Copolymerisates

1060 g eines wässrigen Gemisches enthaltend 530 g monodisperse microverkapselte Monomertröpfchen mit einer mittleren Teilchengröße von 320 µm und einem Ø (90)/Ø (10)-Wertvon 1,03, bestehend aus 94,53 Gew.-% Styrol, 4,98 Gew.-% Divinylbenzol und 0,50 Gew.% tert-Butylperoxy-2-ethylhexanoat werden mit einer wässrigen Lösung aus 1,79 g Gelatine, 2,97 g Natriumhydrogenphosphatdodekahydrat und 148 mg Resorcin in 1177 ml entionisiertem Wasser in einem 4 1 Glasreaktor versetzt. Die Mischung wird unter Rühren (Rührgeschwindigkeit 200 Upm) 8 h bei 75°C und anschließend 2 h bei 95°C polymerisiert. Der Ansatz wird über ein 32 µm-Sieb gewaschen und getrocknet. Man erhält 524 g eines Perlpolymerisats mit glatter Oberfläche. Die Polymerisate erscheinen optisch transparent.

521,7 g eines monodispersen mikroverkapselten Polymerisats, hergestellt nach obigem Verfahren, werden mit einer wässrigen Lösung von 3,58 g Borsäure und 0,98 g Natriumhydroxid in 522 g entionisiertem Wasser versetzt. Zu dieser Mischung wird unter Rühren (Rührgeschwindigkeit 220 Upm) ein Gemisch aus 135,2 g Styrol, 22,4 g 80,6 %igem Divinylbenzol, 12,0 g Acrylnitril, 0,61 g tert-Butylperoxy-2-ethylhexanoat und 0,42 g tert-Butylperoxybenzoat (Zugabegeschwindigkeit: 4,0 g/min) gegeben. Nach einer Einquellzeit von 1 h wird das Gemisch mit einer wässrigen Lösung von 2,88 g Methylhydroxyethylcellulose (Walocel MT 400® ) in 144 g entionisiertem Wasser versetzt. Die Mischung wird 10 h bei 63°C polymerisiert. Unmittelbar nach Erreichen der Polymerisationstemperatur von 63°C wird ein Monomergemisch aus 405,5 g Styrol, 67,3 g 80,6 %igem Divinylbenzol, 36,0 g Acrylnitril, 1,83 g tert-Butylperoxy-2-ethylhexanoat und 1,27 g tert-Butylperoxybenzoat in Form einer Emulsion in entionisiertem Wasser über einen Zeitraum von 5 h mit einer gleichbleibenden Geschwindigkeit zugetropft. Das Monomergemisch wird mit Hilfe eines Rotor-Stator-Rührers in einer Lösung aus 2,57 g ethoxyliertes Nonylphenol (Arkopal N 060® ) und 1,71 g Sulfobernsteinsäureisooctylester-Natriumsalz (75 Gew.-%ig in Ethanol) in 780 g entionisiertem Wasser emulgiert (Größe der emulgierten Monomertröpfchen: 1 bis 2 µm). Anschließend wird der Ansatz 3 h bei 130°C gehalten. Der Ansatz wird über ein 32 µm-Sieb gewaschen und getrocknet. Man erhält 1192 g eines Perlpolymerisats mit glatter Oberfläche. Die Polymerisate erscheinen optisch transparent; die mittlere Teilchengröße beträgt 420 µm und der ⌀ (90)/⌀ (10)-Wert beträgt 1,04.

### b) Herstellung eines Kationenaustauschers

In einem 2 1 Vierhalskolben werden 1800 ml 97,32 Gew.-%ige Schwefelsäure vorgelegt und auf 100°C erhitzt. In 4 h werden in 10 Portionen - insgesamt 400 g trockenes Polymerisat aus Beispiel 2a, unter Rühren eingetragen. Anschließend wird weitere 6 h bei 120°C gerührt. Nach dem Abkühlen wird die Suspension in eine Glassäule überführt. Schwefelsäuren abnehmender Konzentrationen, beginnend mit 90 Gew.-%, zuletzt reines Wasser werden von oben über die Säule filtriert. Man erhält 1790 ml Kationenaustauscher in der H-Form.

### Beispiel 3 (erfindungsgemäß)

### a) Herstellung eines Copolymerisates

1040,2 g eines wäßrigen Gemisches enthaltend 520,1 g monodisperse microverkapselte Monomertröpfchen mit einer mittleren Teilchengröße von 340 µm und einem Ø (90)/Ø (10)-Wert von 1,03, bestehend aus 96,52 Gew.-% Styrol, 2,99 Gew.-% Divinylbenzol und 0,50 Gew.-% tert-Butylperoxy-2-ethylhexanoat werden mit einer wässrigen Lösung aus 2,56 g Gelatine, 4,22 g Natriumhydrogenphosphatdodekahydrat und 212 mg Resorcin in 980,4 ml entionisiertem Wasser in einem 4 1 Glasreaktor versetzt. Zu dem Gemisch werden unter Rühren (Rührgeschwindigkeit 200 Upm) 45,6 g Acrylnitril gegeben. Die Mischung wird 8 h bei 75°C und anschließend 2 h bei 95°C polymerisiert. Der Ansatz wird über ein 32 µm-Sieb gewaschen und getrocknet. Man erhält 1036 g eines Perlpolymerisats mit glatter Oberfläche. Die Polymerisate erscheinen optisch transparent. Das Polymerisat enthält 3,8 Gew.% Acrylnitril (Elementaranalyse).

521,7 g eines monodispersen mikroverkapselten Polymerisats, hergestellt nach obigem Verfahren, werden mit einer wässrigen Lösung von 3,58 g Borsäure und 0,98 g Natriumhydroxid in 1100,0 g entionisiertem Wasser versetzt. Zu dieser Mischung wird unter Rühren (Rührgeschwindigkeit 220 Upm) ein Gemisch aus 109,4 g Styrol, 20,7 g 80,6 %igem Divinylbenzol, 5,6 g Acrylnitril, 0,49 g tert-Butylperoxy-2-ethylhexanoat und 0,34 g tert-Butylperoxybenzoat (Zugabegeschwindigkeit: 4,0 g / min) gegeben. Nach einer Einquellzeit von 1 h wird das Gemisch mit einer wässrigen Lösung von 2,44 g Methylhydroxyethylcellulose (Walocel MT 400® ) in 122 g entionisiertem Wasser versetzt. Die Mischung wird 10 h bei 63°C polymerisiert. Unmittelbar nach Erreichen der Polymerisationstemperatur von 63°C wird ein Monomergemisch aus 437,4 g Styrol, 82,6 g 80,6 %igem Divinylbenzol, 22,6 g Acrylnitril, 1,95 g tert-Butylperoxy-2-ethylhexanoat und 1,36 g tert-Butylperoxybenzoat über einen Zeitraum von 5 h mit einer gleichbleibenden Geschwindigkeit zugetropft. Anschließend wird der Ansatz 3 h bei 130°C gehalten. Der Ansatz wird über ein 32 µm-Sieb gewaschen und getrocknet. Man erhält 1185 g eines Perlpolymerisats mit glatter Oberfläche. Die Polymerisate erscheinen optisch transparent; die mittlere Teilchengröße beträgt 420 µm und der ⌀ (90)/Ø (10)-Wert beträgt 1,06.

### b) Herstellung eines Kationenaustauschers

In einem 2 l Vierhalskolben werden 1800 ml 97,32 gew.% ige Schwefelsäure vorgelegt und auf 100°C erhitzt. In 4 h werden in 10 Portionen - insgesamt 400 g trockenes Polymerisat aus Beispiel 3a, unter Rühren eingetragen. Anschließend wird weitere 6 h bei 120°C gerührt. Nach dem Abkühlen wird die Suspension in eine Glassäule überführt. Schwefelsäuren abnehmender Konzentrationen, beginnend mit 90 Gew.%, zuletzt reines Wasser werden von oben über die Säule filtriert. Man erhält 1794 ml Kationenaustauscher in der H-Form.

## Patentansprüche

1. Verfahren zur Herstellung von monodispersen gelförmigen Kationenaustauschern mit erhöhter Oxidationsstabilität und hoher osmotischer Stabilität und Reinheit, **dadurch gekennzeichnet, dass** man
a) monodisperse microverkapselte Monomertröpfchen aus einem Monomergemisch 1 enthaltend 90,5 bis 97,99 Gew.-% Styrol, 2 bis 7 Gew.-% Divinylbenzol und 0,01 bis 2,5 Gew.-% Radikalbildner in wässriger Suspension bis zu Umsätzen von 76 - 100 % polymerisiert,
b) ein Monomergemisch 2 aus 70,5 bis 95,99 Gew.-% Styrol, 4 bis 15 Gew.-% Divinylbenzol, 0 bis 12 Gew.-% eines dritten Comonomers und 0,01 bis 2,5 Gew.-% eines oder meherer Radikalbildner unter Bildung eines Copolymerisates zusetzt, wobei man eine Teilmenge von 50 bis 100 Gew.-% des Monomergemisches 2 unter polymerisierenden Bedingungen zusetzt, unter denen mindestens ein Radikalbildner aus Monomergemisch 2 aktiv ist, und schließlich
c) das Reaktionsprodukt aus Verfahrensschritt b) durch Sulfonierung funktionalisiert.

2. Verfahren gemäß Anspruch 1, **dadurch gekennzeichnet, dass** man nach Verfahrensschritt b) in einem Zwischenschritt b') den Polymerisationsumsatz der Monomerengemische 1 und 2 erhöht und das Reaktionprodukt schließlich durch Sulfonierung funktionalisiert.

3. Verfahren nach Anspruch 1 oder 2, **dadurch gekennzeichnet, dass** die wässrige Suspension im Verfahrensschritt a) 1 bis 10 Gew.-% Acrylnitril bezogen auf Monomergemisch 1 enthält.

4. Verfahren nach Anspruch 1 bis 3, **dadurch gekennzeichnet, dass** man das Monomergemisch 2 zu dem aus Verfahrensschritt a) erhaltenen Polymerisat in der Weise zusetzt, dass eine Teilmenge von 60 bis 90 Gew.-% unter Bedingungen zugesetzt wird, unter denen mindestens einer der Radikalbildner aus Monomergemisch 2 aktiv ist.

5. Verfahren nach Anspruch 1 bis 4, **dadurch gekennzeichnet, dass** die Zugabe der Teilmenge des Monomergemisches 2, die unter polymerisierenden Bedingungen zugesetzt wird, über einen Zeitraum von 10 bis 1000 min, vorzugsweise von 30 bis 600 min erfolgt.

6. Verfahren nach Anspruch 1 bis 5, **dadurch gekennzeichnet, dass** das Monomergemisch 2 im Verfahrensschritt b) zumindest teilweise als Emulsion in Wasser zugesetzt wird.

7. Verfahren nach Anspruch 1 bis 6, **dadurch gekennzeichnet, dass** als Radikalbildner im Monomergemisch 1 und / oder 2 ein aliphatischer Peroxyester der Formeln 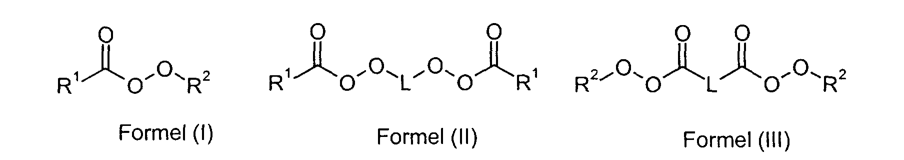 worin
R¹ für einen Alkylrest mit 2 bis 20 C-Atomen oder einen Cycloalkylrest mit bis zu 20 C-Atomen steht,
R² für einen verzweigten Alkylrest mit 4 bis 12 C-Atomen steht und
L für einen Alkylenrest mit 2 bis 20 C-Atomen oder einen Cycloalkylenrest mit bis zu 20 C-Atomen steht,
eingesetzt wird.

8. Verfahren nach Anspruch 1 bis 7, **dadurch gekennzeichnet, dass** die Sulfonierung ohne Quellungsmittel erfolgt.

9. Kationenaustauscher in der H-Form erhältlich nach einem der Ansprüche 1 bis 8.

10. Kationenaustauscher in der Natrium-Form, **dadurch gekennzeichnet, dass** man die Kationenaustauscher gemäß Anspruch 9 mit Natronlauge einer Konzentration von 10 bis 60 Gew.-% umläd.

11. Verwendung der Kationenaustauscher gemäß der Ansprüche 9 und 10 bei der Trinkwasseraufbereitung, Herstellung von Reinstwasser, chromatografischen Trennung von Zuckern oder als Katalysatoren für chemische Reaktionen.

12. Verwendung der Kationenaustauscher gemäß der Ansprüche 9 und 10 zur Bisphenol-A-Synthese aus Phenol und Aceton.
